# EUROPÄISCHE PATENTSCHRIFT

(11) **EP 1 304 994 B1**
(45) Veröffentlichungstag und Bekanntmachung des Hinweises auf die Patenterteilung: **20.10.2004**
(21) Anmeldenummer: 01962863.5
(22) Anmeldetag: 19.07.2001
(51) Int. Cl.: A61K 7/13, C07D 207/32

(54) **PYRROLYLSUBSTITUIERTE NITROPHENOLE ALS DIREKTFARBSTOFFE**
PYRROLYL-SUBSTITUTED NITROPHENOLS USED AS DIRECT COLORANTS
NITROPHENOLS SUBSTITUES PYRROLYLE EN TANT QUE COLORANTS DIRECTS

(30) Priorität: 28.07.2000 DE 10036748
(43) Veröffentlichungstag der Anmeldung: 02.05.2003
(73) Patentinhaber: Henkel Kommanditgesellschaft auf Aktien, 40589 Düsseldorf (DE)
(72) Erfinder: MEINIGKE, Bernd, 51381 Leverkusen (DE); ROSE, David, 40723 Hilden (DE)
(86) Internationale Anmeldenummer: PCT/EP2001/008362
(87) Internationale Veröffentlichungsnummer: WO 2002/009661

(56) Entgegenhaltungen:
- EP-A- 0 170 069
- EP-A- 0 963 982
- US-A- 5 019 130
- CAMPIANI ET AL.: "synthesis, biological activity, and SARs of pyrrolobenzoxazepine derivatives, a new class of specific "peripheral-type" benzodiazepine receptor ligands" J.MED.CHEM., Bd. 39, Nr. 18, 1996, Seiten 3435-3450, XP001042312

## Beschreibung

Gegenstand der Erfindung sind Haarfärbemittel mit einem Gehalt an speziellen direktziehenden Haarfarbstoffen.

Für das Färben von Haar spielen neben den Oxidationsfarbstoffen, die durch oxidative Kupplung einer oder mehrerer Entwicklerkomponenten untereinander oder mit einer oder mehreren Kupplerkomponenten entstehen, besonders die direktziehenden Haarfarbstoffe eine wichtige Rolle. Die direktziehenden Farbstoffe haben den Vorteil, daß sie ohne Zusatz von Oxidationsmitteln zur Anwendung kommen. Als direktziehende Farbstoffe werden vorwiegend Verbindungen eingesetzt, die zur Gruppe der Nitrobenzolderivate gehören. Sie werden entweder allein oder in Kombination mit anderen direktziehenden Farbstoffen wie Anthrachinonfarbstoffen, Indophenolen, Triphenylmethanfarbstoffen, kationischen Azofarbstoffen oder mit Oxidationsfarbstoffen eingesetzt.

Üblicherweise enthalten Haarfärbemittel solche direktziehende Haarfarbstoffe in einem kosmetischen Träger. Als kosmetische Träger für die direktziehenden Haarfarbstoffe und die zur Erzielung bestimmter Nuancen gegebenenfalls im Haarfärbemittel zusätzlich enthaltenen Oxidationsfarbstoff-vorprodukte dienen Cremes, Emulsionen, Gele, Shampoos, Schaumaerosole oder andere Zubereitungen, die für die Anwendung auf dem Haar geeignet sind.

Gute Haarfärbemittel müssen die gewünschten Farbnuancen in ausreichender Intensität ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Reibung und den Einfluß chemischer Reduktionsmittel, z.B. Dauerwellenflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Direktziehende Farbstoffe sollen weiterhin eine gute Verträglichkeit mit anderen Farbstoffen, z.B. mit Oxidationsfarbstoffvorprodukten und mit den in Oxidationshaarfärbemitteln üblichen Komponenten aufweisen, da man zur Nuancenabwandlung gerne direktziehende Farbstoffe und Oxidationsfarbstoffe kombiniert. Daher ist eine gute Stabilität gegen Reduktionsmittel und gegen Oxidationsmittel erforderlich.

Unter den direktziehenden Nitrobenzolderivaten spielen die Nitroaminophenole eine besondere Rolle, da einige dieser Farbstoffe intensive Färbungen von guter Lichtechtheit ausbilden. Solche Farbstoffe sind z.B. aus DE 33 028 17 A1 oder DE 40 183 35 A1 bekannt. Leider ist das Aufziehvermögen und die Waschechtheit solcher Direktfarbstoffe noch nicht in jeder Hinsicht befriedigend.

Es wurde nunmehr gefunden, daß pyrrolylsubstituierte Nitrophenole sich besonders gut als direktziehende Farbstoffe zur Färbung von Fasern eignen, da sie intensive Färbungen in brillianten gelben bis orangefarbenen Nuancen ausbilden. Gegenstand der Erfindung ist daher die Verwendung von pyrrolylsubstituierten Nitrophenolen der Formel (I) 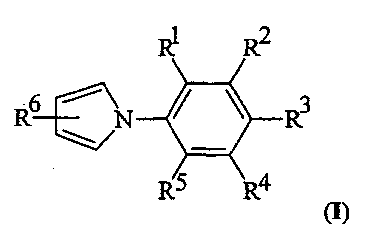 in der
- R¹ steht für Wasserstoff oder eine Hydroxygruppe,
- R² steht für Wasserstoff, ein Halogenatom, eine C₁-C₄-Alkylgruppe, eine C₁-C₄-Alkoxygruppe, eine Nitrogruppe oder eine Sulfonylgruppe
- R³ steht für Wasserstoff, eine C₁-C₄-Alkylgruppe, eine C₁-C₄-Alkoxygruppe, eine Sulfonylgruppe, eine Hydroxygruppe oder eine Nitrogruppe,
- R⁴ und R⁵ stehen unabhängig voneinander für Wasserstoff, eine C₁-C₄-Alkylgruppe, eine C₁-C₄-Alkoxygruppe, eine Nitrogruppe oder eine Sulfonylgruppe,
- und R⁶ steht für Wasserstoff; ein Halogenatom, eine C₁-C₄-Alkylgruppe oder eine C₁-C₄-Alkoxygruppe,
mit der Maßgabe, daß nur eine der Gruppen R², R³, R⁴ oder R⁵ für eine Nitrogruppe steht und daß nur eine der Gruppen R¹ oder R³ für eine Hydroxygruppe steht,
in einem wässrigen Träger zur Färbung von Fasern, insbesondere von Keratinfasern.

In einer bevorzugten Ausführungsform der Verbindungen gemäß Formel (I) steht einer der Substituenten R², R³, R⁴ oder R⁵ für eine Nitrogruppe und die übrigen dieser Substituenten für Wasserstoff.

In einer weiteren bevorzugten Ausfuhrungsform der Verbindungen gemäß Formel (I) steht R² für Wasserstoff oder Chlor.

Des weiteren ist es bevorzugt Verbindungen gemäß Formel (I) zu verwenden, in denen R⁶ steht für Wasserstoff.

Es ist erfindungsgemäß bevorzugt, die Verbindungen gemäß Formel I ausgewählt aus 4-Nitro-2-(1H-pyrrol-1-yl)-phenol, 3-Nitro-4-(1H-pyrrol-1-yl)-phenol, 2-Nitro-4-(1Hpyrrol-1-yl)-phenol, 4-Nitro-6-chlor-2-(1H-pyrrol-1-yl)-phenol, 3-Nitro-6-(1H-pyrrol-1-yl)-phenol zu verwenden.

Beispiele für die als Substituenten im Rahmen dieser Anmeldung genannten C₁-C₄-Alkylreste sind die Gruppen Methyl, Ethyl, Propyl, Isopropyl und Butyl. Ethyl und Methyl sind bevorzugte Alkylreste. Beispiele für bevorzugte C₂-C₄-Alkenylreste sind Vinyl und Allyl. Erfindungsgemäß bevorzugte C₁-C₄-Alkoxyreste sind beispielsweise eine Methoxy- oder eine Ethoxygruppe. Weiterhin können als bevorzugte Beispiele für eine C₁-C₄-Monohydroxyalkylgruppe eine Hydroxymethyl-, eine 2-Hydroxyethyl-, eine 3-Hydroxypropyl- oder eine 4-Hydioxybutylgrappe genannt werden. Eine 2-Hydroxyethylgruppe ist besonders bevorzugt. Ein Beispiel für eine bevorzugte C₂-C₄-Polyhydroxyalkylgruppe ist die α,β-Dihydroxyethylgruppe. Beispiele für Halogenatome sind erfindungsgemäß F-, Cl-, Br oder I-Atome, Cl-Atome sind ganz besonders bevorzugt. Die weiteren verwendeten Begriffe leiten sich erfindungsgemäß von den hier gegebenen Definitionen ab. Die in diesem Abschnitt getroffenen Definitionen gelten auch für die Formeln (E1), (E2), (E3), (E4) und (IIa) sowie (IIb).

Die erfindungsgemäß geeigneten pyrrolylsubstituierten Nitrophenole sind teilweise literaturbekannt. Neu und ein weiterer Gegenstand der Erfindung sind die Verbindungen der Formel (1), die durch die Substituentenkombination A und B gekennzeichnet sind:

| | **R**^{**1**} | **R**^{**2**} | **R**^{**3**} | **R**^{**4**} | **R**^{**5**} | **R**^{**6**} |
|---|---|---|---|---|---|---|
| A | H | -NO₂ | -OH | H | H | H |
| B | -OH | Cl | H | -NO₂ | H | H |

Diese Verbindungen sind aus dem entsprechenden Amino-nitrophenol durch Umsetzung mit 2,5-Dimethoxytetrahydrofuran zugänglich. Synthesevorschriften sind im Beispielteil angegeben.

Die Farbstoffe der Formel (I) erzeugen auf der Faser intensiv gelbe bis orange Farbnuancen mit guten Echtheitseigenschaften. Als Fasern können damit sowohl Cellulosefasern (Baumwolle, Leinen, Jute, Hanf), Regeneratcellulose (Acetatseide, Kupferseide, Viskose), Proteinfasem (Seide, Wolle, Pelze, Federn, Haare) als auch Synthesefasern (Polyamid, Polyester, Polyurethan) gefärbt werden. In dermatologischer und toxikologischer Hinsicht sind die Verbindungen der Formel (I) unschädlich und daher besonders geeignet für die Färbung von menschlichem Haar.

Ein weiterer Gegenstand der Erfindung sind Mittel zum Färben und Tönen von keratinischen Fasern, insbesondere von menschlichen Haaren, die dadurch gekennzeichnet sind, daß als direktziehender Farbstoff wenigstens ein pyrrolylsubstituiertes Nitrophenolderivat der Formel (I) enthalten ist. Es wurde überraschenderweise gefunden, daß sich mit den erfindungsgemäßen Haarfärbemitteln Haaranfärbungen von hoher Intensität und guten Echtheitseigenschaften, besonders auch von guter Waschechtheit und hoher Stabilität gegen Ausbluten und Farbveränderungen beim Shamponieren erzielen lassen.

Die erfindungsgemäßen Haarfärbemittel können die direktziehenden Verbindungen gemäß Formel (I) allein oder in Kombination mit bekannten direktziehenden Farbstoffen, z.B. mit anderen Nitrobenzolderivaten, Anthrachinonfarbstoffen, Triphenylmethan- oder Azofarbstoffen enthalten.

Bevorzugt enthalten die erfindungsgemäßen Haarfärbemittel zur weiteren Modifizierung der Farbnuancen zusätzlich folgende übliche direktziehende Farbstoffe: Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 12, Disperse Blue 3, HC Violet 1, Disperse Violet 1, Disperse Violet 4, Acid Violet 43, Disperse Black 9 und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

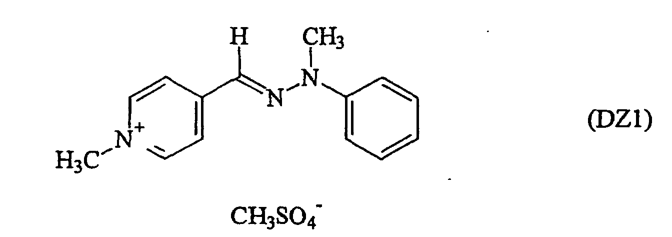

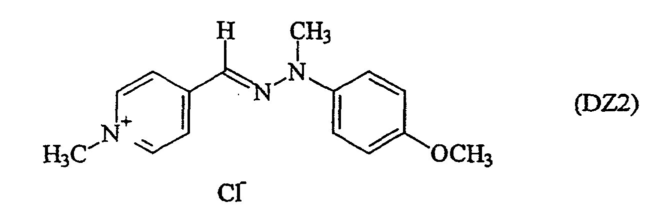

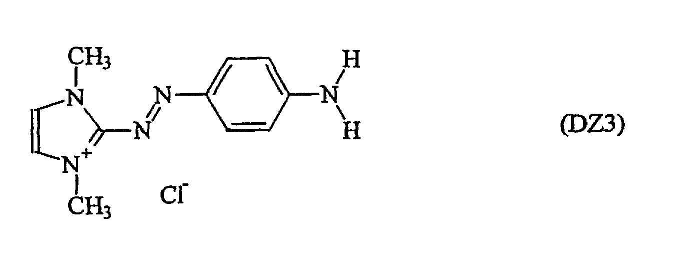

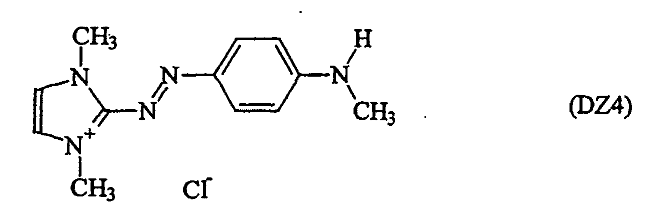

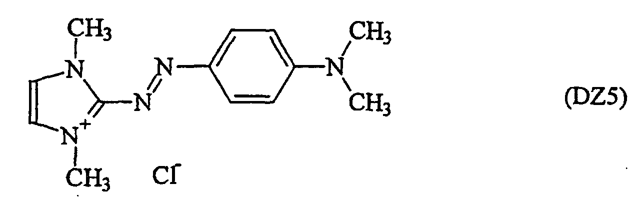

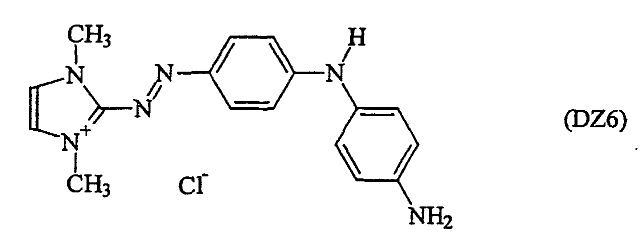

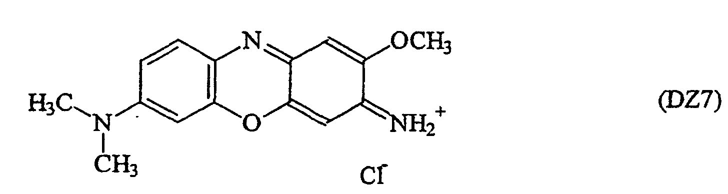

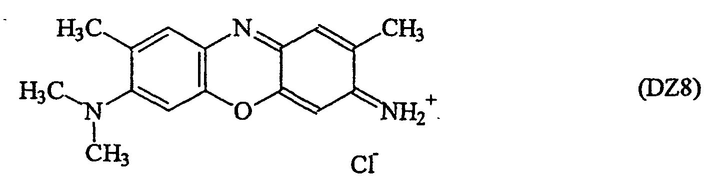

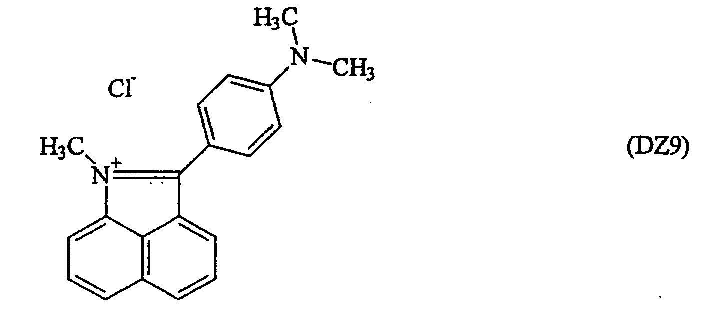

Die Verbindungen der Formeln (DZ1), (DZ3) und (DZ5) sind ganz besonders bevorzugte kationische direktziehende Farbstoffe der Gruppe (c). Ebenso sind die kationischen direktziehenden Farbstoffe, die unter dem Warenzeichen Arianor® vertrieben werden, erfindungsgemäß ganz besonders bevorzugte direktziehende Farbstoffe.

Die erfindungsgemäßen Mittel gemäß dieser Ausβihmngsformen enthalten die direktziehenden Farbstoffe bevorzugt in einer Menge von 0,01 bis 20 Gew.-%, bezogen auf das gesamte Färbemittel.

Darüber hinaus eignen sich die direktziehenden Farbstoffe gemäß Formel (I) wegen ihrer hohen Beständigkeit gegenüber Reduktionsmitteln und Oxidationsmitteln auch sehr gut zur Kombination mit Oxidationsfarbstoff-vorprodukten, also zur Modifikation der Nuancen von Oxidationshaarfärbemitteln. Oxidationshaarfärbemittel enthalten als Farbstoffvorprodukte Entwicklerkomponenten, die durch oxidative Kupplung untereinander oder mit geeigneten Kupplerkomponenten die Oxidationsfarbstoffe ausbilden. Als Entwicklersubstanzen werden z.B. primäre aromatische Amine mit einer weiteren, in Paraoder Orthoposition befindlichen freien oder substituierten Hydroxy- oder Aminogruppe, Diaminopyridinderivate, heterocyclische Hydrazone, 4-Aminopyrazolonderivate und 2,4,5,6-Tetraaminopyrimidin und dessen Derivate eingesetzt.

Es kann erfindungsgemäß bevorzugt sein, als eine Entwicklerkomponente ein p-Phenylendiaminderivat oder eines seiner physiologisch verträglichen Salze einzusetzen. Besonders bevorzugt sind p-Phenylendiaminderivate der Formel (E1) 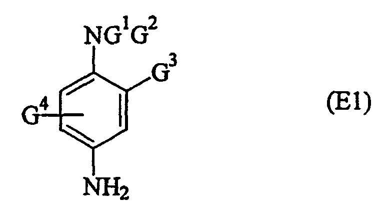 wobei
- G¹ steht für ein Wasserstoffatom, einen C₁-C₄-Alkykest, einen C₁-C₄-Monohydroxyalkylrest, einen C₂-C₄-Polyhydroxyalkylrest, einen (C₁-C₄)-Alkoxy-(C₁-C₄)-alkylrest, einen 4'-Aminophenylrest oder einen C₁-C₄-Alkylrest, der mit einer stickstoffhaltigen Gruppe, einem Phenyl- oder einem 4'-Aminophenylrest substituiert ist,
- G² steht für ein Wasserstoffatom, einen C₁-C₄-Alkylrest, einen C₁-C₄-Monohydroxyalkylrest, einen C₂-C₄-Polyhydroxyalkylrest, einen (C₁-C₄)-Alkoxy-(C₁-C₄)-alkylrest oder einen C₁-C₄-Alkylrest, der mit einer stickstoffhaltigen Gruppe substituiert ist,
- G³ steht für ein Wasserstoffatom, ein Halogenatom, wie ein Chlor-, Brom-, Iod- oder Fluoratom, einen C₁-C₄-Alkylrest, einen C₁-C₄-Monohydroxyalkylrest, einen C₂-C₄-Polyhydroxyalkylrest, einen C₁-C₄-Hydroxyalkoxyrest, einen C₁-C₄-Acetylaminoalkoxyrest, einen C₁-C₄-Mesylaminoalkoxyrest oder einen C₁-C₄-Carbamoylaminoalkoxyrest,
- G⁴ steht für ein Wasserstoffatom, ein Halogenatom oder einen C₁-C₄-Alkylrest oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Beispiele für stickstoffhaltige Gruppen der Formel (E1) sind insbesondere die Aminogruppen, C₁-C₄-Monoalkylaminogruppen, C₁-C₄-Dialkylaminogruppen, C₁-C₄-Trialkylammoniumgruppen, C₁-C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-pphenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-Hydroxyethyl)amino-2-chloranilin, 2-(β-Hydroxyethyl)-pphenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenylp-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Aminound/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 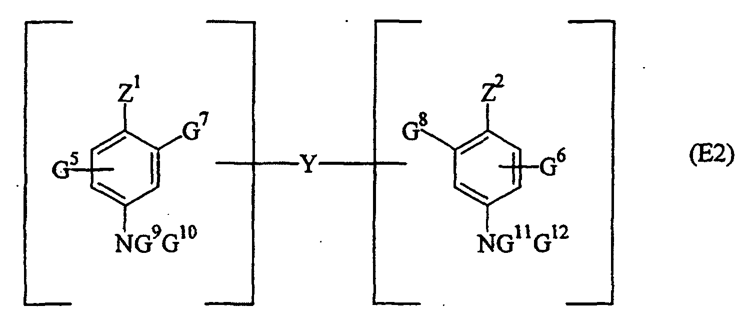 wobei:
- Z¹ und Z² stehen unabhängig voneinander für einen Hydroxyl- oder NH₂-Rest, der gegebenenfalls durch einen C₁-C₄-Alkylrest, durch einen C₁-C₄-Monohydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁-C₈-Alkoxyreste substituiert sein kann, oder eine direkte Bindung,
- G⁵ und G⁶ stehen unabhängig voneinander für ein Wasserstoff- oder Halogenatom, einen C₁-C₄-Alkylrest, einen C₁-C₄-Monohydroxyalkylrest, einen C₂-C₄- Polyhydroxyalkylrest, einen C₁-C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁-C₄-Alkylrest,
mit den Maßgaben, daß
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetra-methylendiamin, N,N'-Bis-(β-hydroxyethyl)-N,N-bis-(4-aminophenyl)-tetramethylendiamin, N,N'-Bis-(4-methyl-aminophenyl)-tetramethylendiamin, N,N'-Bis-(ethyl)-N,N'-bis-(4'-amino-3'-methylphenyl)-ethylendiamin, Bis-(2-hydroxy-5-aminophenyl)-methan, 1,4-Bis-(4'-aminophenyl)-d.iazacycloheptan, N,N-Bis-(2-hydroxy-5-aminobenzyl}-piperazin, N-(4'-Aminophenyi)-pphenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 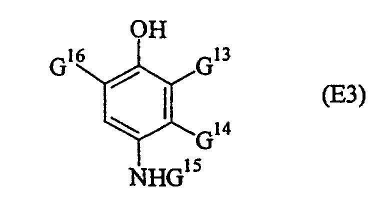 wobei
- G¹³ steht für ein Wasserstoffatom, ein Halogenatom, einen C₁-C₄-Alkylrest, einen C₁-C₄-Monohydroxyalk-ylrest, einen C₂-C₄-Polyhydroxyalkylrest, einen (C₁-C₄)-Alkoxy-(C₁-C₄)-alkylrest, einen C₁-C₄-Aminoalkylrest, einen Hydroxy-(C₁-C₄)-alkylaminorest, einen C₁-C₄-Hydroxyalkoxyrest, einen C₁-C₄-Hydroxyalkyl-(C₁-bis C₄)-aminoalkylrest oder einen (Di-C₁-C₄-Alkylamino)-(C₁-C₄)-alkylrest, und
- G¹⁴ steht für ein Wasserstoff- oder Halogenatom, einen C₁-C₄-Alkylrest, einen C₁-C₄-Hydroxyalkylrest, einen C₂-C₄-Polyhydroxyalkyirest, einen (C₁- C₄)-Alkoxy-(C₁-C₄)-alkylrest, einen C₁-C₄-Aminoalkylrest oder einen C₁- C₄-Cyanoalkylrest,
- G¹⁵ steht für Wasserstoff, einen C₁-C₄-Alkylrest, einen C₁-C₄-Hydroxyalkylrest, einen C₂-C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-Aminophenol, 4-Amino-3-methylphenol, 4-Amino-3-fluorphenol, 2-Hydroxymethylamino-4-arninophenol, 4-Amino-3-hydroxymethylphenol, 4-Amino-2-(2-hydroxyethoxy)phenol, 4-Amino-2-methylphenol, 4-Amino-2-hydroxymethylphenol, 4-Amino-2-methoxymethylphenol, 4-Amino-2-aminomethylphenol, 4-Amino-2-(β-hydroxyethyl-aminomethyl)phenol, 4-Amino-2-fluorphenol, 4-Amino-2-chlorphenol, 2,6-Dichlor-4-aminophenol, 4-Amino-2-((diethylamino)methyl)phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol und 4-Amino-2-((diethylamino)methyl)phenol.

Ferner kann die Entwicklerkomponente ausgewählt sein aus o-Aminophenol und seinen Derivaten, wie beispielsweise 2-Amino-4-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen. Bevorzugt werden erfindungsgemäß Pyrimidin oder Pyrazolderivate.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'chlorobenzyl)-pyrazol, 4,5-Diamino-1,3-dimethylpyrazol, 4,5-Diamino-3-methyl-1phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamino-6-methoxy-pyridin, 2-(β-Methoxyethyl)amino-3-amino-6-methoXy-pyridin und 3,4-Diamino-pyridin.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 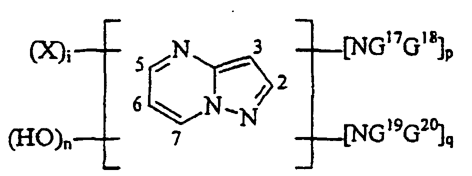 wobei:
- G¹⁷, G¹⁸, G¹⁹ und G²⁰ unabhängig voneinander stehen für ein Wasserstoffatom, einen C₁-C₄-Alkylrest, einen Aryl-Rest, einen C₁-C₄-Hydroxyalkylrest, einen C₂-C₄-Polyhydroxyalkylrest einen (C₁-C₄)-Alkoxy-(C₁-C₄)-alkylrest, einen C₁-C₄-Aminoalkylrest, der gegebenenfalls durch einen Acetyl-Ureid- oder Sulfonyl-Rest geschützt sein kann, einen (C₁-C₄)-Alkylamino-(C₁-C₄)-alkylrest, einen Di-[(C₁-C₄)-alkyl]-(C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroxyalkyl- oder einen Di-(C₁-C₄)-[Hydroxyalkyl]-(C₁-C₄)-aminoalkylrest,
- die X-Reste stehen unabhängig voneinander für ein Wasserstoffatom einen C₁-C₄-Alkylrest, einen Aryl-Rest, einen C₁-C₄-Hydroxyalkylrest, einen C₂-C₄- Polyhydroxyalkylrest, einen C₁-C₄-Aminoalkylrest, einen (C₁-C₄)-Alkylamino-(C₁-C₄)-alkylrest, einen Di-[(C₁-C₄)alkyl]- (C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroxyalkyl- oder einen Di-(C₁-C₄-hydroxyalkyl)-aminoalkylrest, einen Aminorest, einen C₁-C₄-Alkyl- oder Di-(C₁-C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

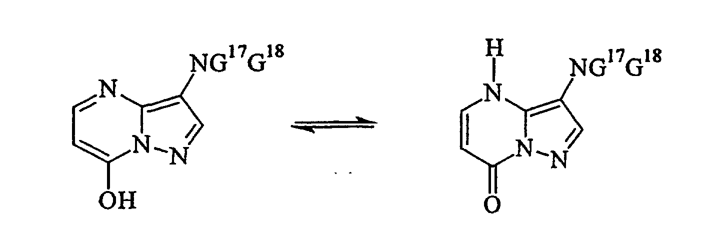

Unter den Pyrazol-[1,5-a] pyrimidinen der obenstehenden Formel (E4) kann man insbesondere nennen:
- Pyrazol-[1,5-a]-pyrimidin-3,7-diamin;
- 2,5-Dimethyl pyrazol-[1,5-a]-pyrimidin-3,7-diamin;
- Pyrazol-(1,5-a]-Pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5-a]-pyrimidin-3,7-diamin;
sowie ihre physiologisch verträglichen Salze und ihre tautomeren Formen, wenn ein tautomeres Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Ganz besonders bevorzugte Entwicklerkomponenten sind erfindungsgemäß ausgewählt aus p-Phenylendiamin, p-Toluylendiamin, p-Aminophenol, 1-(2'-Hydroxyethyl)-2,5-diaminobenzol, N,N-Bis-(2'-hydroxyethyl)-p-phenylendiamin, 4-Amino-3-methylphenol, 4-Amino-2-((diethylamino)-methyl)-phenol, 2-Aminomethyl-4-aminophenol, 2,4,5,6-Tetraaminopyrimidin, 2-Hydroxy-4,5,6-triaminopyrimidin, 4-hydroxy-2,5,6-triaminopyrimidin und 4,5-Diamino-1-(2'-hydroxyethyl)-pyrazol.

Als Kupplersubstanzen werden beispielsweise m-Phenylendiaminderivate, Naphthole, Resorcin und Resorcinderivate, Pyrazolone und m-Aminophenole verwendet.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoracetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)propan, 1-Methoxy-2-amino-4-(2'-hydroxyethylamino)-benzol, 1,3-Bis-(2',4'-diaminophenyl)propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Aniino-5-chlor-3-hydroxypyridm, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin,- 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioaybenzol.

Besonders bevorzugt werden erfindungsgemäß 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-dimethylpyridin als Kupplerkomponenten verwendet.

In den erfindungsgemäßen Haarfärbemitteln sind die direktziehenden Haarfarbstoffe der allgemeinen Formel (I) in einer Menge von 0,01 bis 5,0 Gew.-%, bevorzugt von 0, 1 bis 2 Gew.-%, bezogen auf das gesamte Haarfärbemittel, enthalten. Daneben können bekannte Oxidationshaarfärbemittelvorprodukte (Entwickler- und Kupplersubstanzen) in einer Menge von 0,01 bis 5 Gew.-%, vorzugsweise von 1 bis 3 Gew.-%, enthalten sein.

Eine natürlich erscheinende Haarfarbe kann aus einem Färbevorgang hervorgehen, wenn in dem applizierten Haarfärbemittel zusätzlich Indol- oder Indolinderivate als Vorprodukte naturanaloger Farbstoffe verwendet werden.

In einer weiteren Ausfühnmgsform werden daher zusätzlich bevorzugt Indole und/oder Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (IIa), 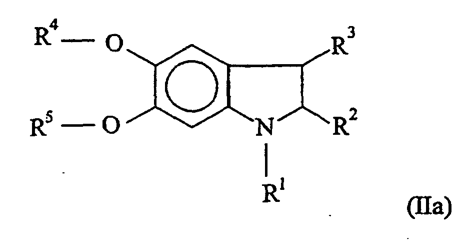 in der unabhängig voneinander
R¹ steht für Wasserstoff, eine C₁-C₄-Alkylgruppe, eine C₃-C₆-Cycloalkylgruppe, eine C₂-C₄-Alkenylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIb),

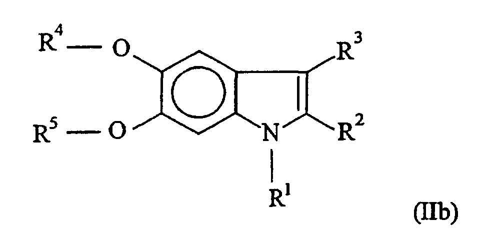

in der unabhängig voneinander
R¹ steht für Wasserstoff, eine C₁-C₄-Alkylgruppe, eine C₃-C₆-Cycloalkylgruppe, eine C₂-C₄-Alkenylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoff-vorprodukte), erschienen als Band 7 der Reihe "Dermatology" (Hrsg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und HandeLsunternehmen für Arzneimittel, Refbrmwaren und Körperpflegemittel e.V., Mannheim, Bezug genommen.

Wenn das erfindungsgemäße Haarfärbemittel Oxidationsfarbstoff-vorprodukte enthält, empfiehlt sich außerdem die Zugabe einer kleinen Menge eines Reduktionsmittels, z.B. von 0,5 bis 2,0 Gew.-% Natriumsulfit zur Stabilisierung der Oxidationsfarbstoffvorprodukte. In diesem Falle wird vor der Anwendung des Haarfärbemittels dieses mit einem Oxidationsmittel versetzt, um die oxidative Entwicklung der Oxidationsfarbstoffvorprodukte einzuleiten. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxydisulfat in Frage.

Zur Herstellung der erfindungsgemäßen Haarfärbemittel werden die direktziehenden Haarfarbstoffe und gegebenenfalls die Oxidationsfarbstoffvorprodukte in einen geeigneten Träger, z.B. in Cremes, Emulsionen, Gele oder auch tensidhaltige, schäumende Lösungen, z.B. in Shampoos, Schaumaerosole oder andere Zubereitungen eingearbeitet, die für die Anwendung auf dem Haar geeignet sind.

Übliche Bestandteile solcher kosmetischer Zubereitungen sind z.B. Netz- und Emulgiermittel wie anionische, nichtionische oder ampholytische Tenside, z.B. Fettalkoholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel, wie z.B. Methyl- oder Hydroxyethylcellulose, Stärke, Fettalkohole, Paraffinöle, Fettsäuren, ferner Parfümöle und haarpflegende Zusätze, wie z.B. wasserlösliche kationische Polymere, Proteinderivate, UV-Filtersubstanzen, Pantothensäure und Cholesterin. Die Bestandteile der kosmetischen Träger werden zur Herstellung der erfindungsgemäßen Haarfärbemittel in für diese Zwecke üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die erfindungsgemäßen Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel RO-(Z)ₓ. Diese Verbindungen sind durch die folgenden Parameter gekennzeichnet.

Der Alkylrest R' enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R'
- im wesentlichen aus C₈- und C₁₀-Alkylgruppen,
- im wesentlichen aus C₁₂- und C₁₄-Alkylgruppen,
- im wesentlichen aus C₈-C₁₆-Alkylgruppen oder
- im wesentlichen aus C₁₂-C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quatemierte Estersalze von Fettsäuren mit Triethanolamin, quatemierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-PaLmitoyloxyethyI)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80).

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die erfindungsgemäßen Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat®100 (Poly(dimethyldiallylammoniumchlorid)), Merquat®550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quatemierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
   sowie die unter den Bezeichnungen
   - Polyquaternium 2,
   - Polyquaternium 17,
   - Polyquaternium 18 und
   - Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquatemium-2, Polyquaternium 10 und Polyquatemium-22.

Alternativ zu den kationischen Polymeren werden als konditionierende Wirkstoffe zwitterionische oder ampholytische Polymere besonders bevorzugt eingesetzt. Bevorzugte Vertreter sind Octylacrylamid/Methylmethacrylat/tert Butylaminoethylmethacrylatl2-Hydroxypropylmethacrylat Copolymere und insbesondere das Acrylamidopropyltrimethylammoniumchlorid/Acrylat Copolymer.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wakker) sowie Abil®-Quat 3270 und 3272 (Hersteller: Th. Goldschmidt; diquatemäre Polydimethylsiloxane, Quatemium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkemöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder voll synthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- fasersttulcturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie den eingesetzten Mengen dieser Komponenten wird ausdrücklich auf die dem Fachmann bekannten einschlägigen Handbücher, z. B. K. Schrader, Grundlagen und Rezepturen der Kosmetika, 2. Auflage, Hüthig Buch Verlag, Heidelberg, 1989, verwiesen.

Die Anwendung der erfindungsgemäßen Haarfärbemittel kann, unabhängig von der Art der kosmetischen Zubereitung, zum Beispiel als Creme, Gel oder Shampoo, im schwach sauren, neutralen oder alkalischen Milieu erfolgen. Bevorzugt ist die Anwendung der Haarfärbemittel in einem pH-Bereich von 6 bis 10. Die Anwendungstemperaturen können in einem Bereich zwischen 15°C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z.B. ein Färbeshampoo, verwendet wurde.

Die nachfolgenden Beispiele sollen den Erfindungsgegenstand näher erläutern, ohne ihn jedoch hierauf zu beschränken.

### Beispiele

### 1. Herstellungsbeispiele

### 1.1 Herstellung von 2-Nitro-4-(1H-pyrrol-1-yl)-phenol (D3)

Eine Mischung aus 5,0 g 4-Amino-2-nitrophenol, 5,4 g 2,5-Dimethoxytetrahydrofuran und 50 ml konzentrierter Essigsäure wurde 2 Stunden bei 60°C gerührt. Nach dem Einengen wurde der Rückstand aus Ethanol umkristallisiert. Es wurden 2,2 g braune Kristalle mit einem Schmelzpunkt von 84 °C erhalten.

### 1.2 Herstellung von 4-Nitro-6-chlor-2-(1H-pyrrol-1-yl)-phenol (D4)

Die Herstellung erfolgte analog 1.1 ausgehend von 2-Amino-6-chlor-4-nitrophenol. Es wurden gelbe Kristalle mit einem Schmelzpunkt von 105 °C erhalten.

### 2. Haarfärbungen

Es wurden Haarfärbecremes der folgenden Zusammensetzungen herstellt:

| | |
|---|---|
| Lorol® techn.¹ | 10,0 g |
| C₁₂₋₁₄-Fettalkohol+2 EO-Sulfat, Na-Salz (28 %ig) | 25,0 g |
| Wasser | 60,0 g |
| Direktfarbstoff (D1 bis D5) | 1,0 g |
| Ammoniumsulfat | 1,0 g |
| konzentrierte Ammoniak-Lösung (25%ig) | bis pH = 9,5 |
| Wasser | ad 100 g |

| | |
|---|---|
| ¹C₁₂₋₁₈-Fettalkohol (INCI-Bezeichnung: Coconut Alcohol) (COGNIS) | |

Die Bestandteile der Haarfärbecreme wurden der Reihe nach miteinander vermischt Nach Zugabe des direktziehenden Farbstoffs wurde mit konzentrierter Ammoniak-Lösung der pH-Wert der Emulsion auf 9,5 eingestellt, dann wurde mit Wasser auf 100 g aufgefüllt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet. Das Ergebnis der Färbeversuche für die Farbstoffe D1 bis D5 ist der Tabelle I zu entnehmen.

| **Direktfarbstoff:** | | **Nuance des gefärbten Haars** |
|---|---|---|
| **D1** | 4-Nitro-2-(1H-pyrrol-1-yl)-phenol ¹⁾ | gelb |
| **D2** | 3-Nitro-4-(1H-pyrrol-1-yl)-phenol ²⁾ | strohgelb |
| **D3** | 2-Nitro-4-(1H-pyrrol-1-yl)-phenol | grauorange |
| **D4** | 4-Nitro-6-chlor-2-(1H-pyrrol-1-yl)-phenol | zitronengelb |
| **D5** | 3-Nitro-6-(1H-pyrrol-1-yl)-phenol ³⁾ | gelb |

| | | |
|---|---|---|
| ¹⁾ gemäß US 4 035 495 | | |
| ²⁾ gemäß J. Med. Chem. (1999), 42, 4362 | | |
| ³⁾ gemäß Chem. Pharm. Bull. (1995), 43, 1358 | | |

## Patentansprüche

1. Verwendung von pyrrolylsubstituierten Nitrophenolen der Formel (I) 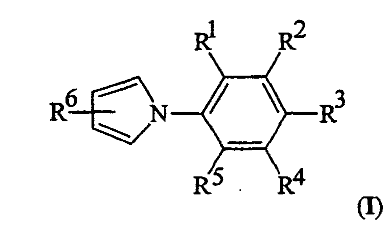 in der
• R¹ steht für Wasserstoff oder eine Hydroxygruppe,
• R² steht für Wasserstoff, ein Halogenatom, eine C₁-C₄-Alkylgruppe, eine C₁-C₄-Alkoxygruppe, eine Nitrogruppe oder eine Sulfonylgruppe
• R³ steht für Wasserstoff, eine C₁-C₄-Alkylgruppe, eine C₁-C₄-Alkoxygruppe, eine Sulfonylgruppe, eine Hydroxygruppe oder eine Nitrogruppe,
• R⁴ und R⁵ stehen unabhängig voneinander für Wasserstoff, eine C₁-C₄-Alkylgruppe, eine C₁-C₄-Alkoxygruppe, eine Nitrogruppe oder eine Sulfonylgruppe,
• und R⁶ steht für Wasserstoff, ein Halogenatom, eine C₁-C₄-Alkylgruppe oder eine C₁-C₄-Alkoxygruppe,
mit der Maßgabe, daß nur eine der Gruppen R², R³, R⁴ oder R⁵ für eine Nitrogruppe steht und daß nur eine der Gruppen R¹ oder R³ für eine Hydroxygruppe steht, in einem wässrigen Träger zur Färbung von Fasern, insbesondere von Keratinfasem.

2. Verwendung von pyrrolylsubstituierten Nitrophenolen nach Anspruch 1, **dadurch gekennzeichnet, daß** einer der Substituenten R², R³, R⁴ oder R⁵ für eine Nitrogruppe steht und die übrigen dieser Substituenten für Wasserstoff stehen.

3. Verwendung von pyrrolylsubstituierten Nitrophenolen nach Anspruch 1, **dadurch gekennzeichnet, daß** R² steht für Wasserstoff oder Chlor.

4. Verwendung von pyrrolylsubstituierten Nitrophenolen nach einem der Ansprüche 1 bis 3, **dadurch gekennzeichnet, daß** R⁶ steht für Wasserstoff.

5. Verwendung von pyrrolylsubstituierten Nitrophenolen nach Anspruch 1, **dadurch gekennzeichnet, daß** die Verbindungen gemäß Formel (I) ausgewählt sind aus 4-Nitro-2-(1H-pyrrol-1-yl)-phenol, 3-Nitro-4-(1H-pyrrol-1-yl)-phenol, 2-Nitro-4-(1Hpyrrol-1-yl)-phenol, 4-Nitro-6-chlor-2-(1H-pyrrol-1-yl)-phenol, 3-Nitro-6-(1Hpyrrol-1-yl)-phenol.

6. Mittel zum Färben und Tönen keratinischer Fasern, insbesondere von menschlichen Haaren, **dadurch gekennzeichnet, daß** es in einem wässrigen Träger als direktziehenden Farbstoff wenigstens ein pyrrolylsubstituiertes Nitrophenol der Formel (I) enthält.

7. Mittel nach Anspruch 6, **dadurch gekennzeichnet, daß** die pyrrolsubstituierten Nitrophenole der Formel (I) in einer Menge von 0,01-5 Gew.-% enthalten sind.

8. Mittel nach einem der Ansprüche 6 oder 7, **dadurch gekennzeichnet, daß** zusätzlich zu den pyrrolsubstituierten Nitrophenolen der Formel (I) mindestens ein weiterer direktziehender Farbstoff und/oder mindestens ein Oxidationsfarbstoff-vorprodukt enthalten ist.

9. Mittel nach einem der Ansprüche 6 bis 8, **dadurch gekennzeichnet, daß** mindestens ein Indol- und/oder Indolinderivat enthalten ist.

10. Mittel nach einem der Ansprüche 6 bis 9, **dadurch gekennzeichnet, daß** der Träger eine Creme, eine Emulsion, ein Gel, ein Shampoo oder ein Schaumaerosol ist.

11. Verbindungen der Formel (I), **gekennzeichnet durch** die folgenden Substituentenkombinationen A und B
| | **R**^{**1**} | **R**^{**2**} | **R**^{**3**} | **R**^{**4**} | **R**^{**5**} | **R**^{**6**} |
|---|---|---|---|---|---|---|
| A | H | -NO₂ | -OH | H | H | H |
| B | -OH | Cl | H | -NO₂ | H | H |

## Claims

1. The use of pyrrolyl-substituted nitrophenols corresponding to formula (I): 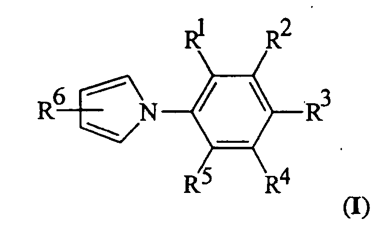 in which
• R¹ is hydrogen or a hydroxy group,
• R² is hydrogen, a halogen atom, a C₁₋₄ alkyl group, a C₁₋₄ alkoxy group, a nitro group or a sulfonyl group,
• R³ is hydrogen, a C₁₋₄ alkyl group, a C₁₋₄ alkoxy group, a sulfonyl group, a hydroxy group or a nitro group,
• R⁴ and R⁵ independently of one another represent hydrogen, a C₁₋₄ alkyl group, a C₁₋₄ alkoxy group, a nitro group or a sulfonyl group and
• R⁶ is hydrogen, a halogen atom, a C₁₋₄ alkyl group or a C₁₋₄ alkoxy group,
with the proviso that only one of the groups R², R³, R⁴ or R⁵ is a nitro group and that only one of the groups R¹ or R³ is a hydroxy group, in a water-containing carrier for colouring fibres, particularly keratin fibres.

2. The use of pyrrolyl-substituted nitrophenols as claimed in claim 1, **characterized in that** one of the substituents R², R³, R⁴ or R⁵ is a nitro group and the other substituents are hydrogen.

3. The use of pyrrolyl-substituted nitrophenols as claimed in claim 1, **characterized in that** R² is hydrogen or chlorine.

4. The use of pyrrolyl-substituted nitrophenols as claimed in any of claims 1 to 3, **characterized in that** R⁶ is hydrogen.

5. The use of pyrrolyl-substituted nitrophenols as claimed in claim 1, **characterized in that** the compounds of formula (I) are selected from 4-nitro-2-(1H-pyrrol-1-yl). -phenol, 3-nitro-4-(1H-pyrrol-1-yl)-phenol, 2-nitro-4-(1H-pyrrol-1-yl)-phenol, 4-nitro-6-chloro-2-(1H-pyrrol-1-yl)-phenol, 3-nitro-6-(1H-pyrrol-1-yl)-phenol.

6. Preparation for colouring and tinting keratinous fibres, more particularly human hair, **characterized in that** it contains at least one pyrrolyl-substituted nitrophenol of formula (I) as substantive dye in a water-containing carrier.

7. A preparation as claimed in claim 6, **characterized in that** the pyrrole-substituted nitrophenols of formula (I) are present in a quantity of 0.01 to 5% by weight.

8. A preparation as claimed in claim 6 or 7, **characterized in that**, in addition to the pyrrole-substituted nitrophenols of formula (I), it contains at least one other substantive dye and/or at least one oxidation dye precursor.

9. A preparation as claimed in any of claims 6 to 8, **characterized in that** it contains at least one indole and/or indoline derivative.

10. A preparation as claimed in any of claims 6 to 9, **characterized in that** the carrier is a cream, an emulsion, a gel, a shampoo or a foam aerosol.

11. A compound of formula (I), **characterized by** the following substituent combinations A and B:
| | **R**^{**1**} | **R**^{**2**} | **R**^{**3**} | **R**^{**4**} | **R**^{**5**} | **R**^{**6**} |
|---|---|---|---|---|---|---|
| A | H | -NO₂ | -OH | H | H | H |
| B | -OH | Cl | H | -NO₂ | H | H |

## Revendications

1. Utilisation de nitrophénols substitués par pyrrolyle de formule (I) 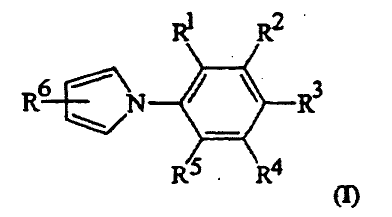 dans laquelle
• R¹ représente hydrogène ou un groupe hydroxy,
• R² représente hydrogène, un atome d'halogène, un groupe alkyle en C₁ à C₄, un groupe alcoxy en C₁ à C₄, un groupe nitro ou un groupe sulfonyle
• R³ représente hydrogène, un groupe alkyle en C₁ à C₄, un groupe alcoxy en C₁ à C₄, un groupe sulfonyle, un groupe hydroxy ou un groupe nitro,
• R⁴ et R⁵ représentent, indépendamment l'un de l'autre, hydrogène, un groupe alkyle en C₁ à C₄, un groupe alcoxy en C₁ à C₄, un groupe nitro ou un groupe sulfonyle,
• et R⁶ représente hydrogène, un atome d'halogène, un groupe alkyle en C₁ à C₄ ou un groupe alcoxy en C₁ à C₄,
à condition que seulement un des groupes R², R³, R⁴ ou R⁵ représente un groupe nitro et que seulement un des groupes R¹ ou R³ représente un groupe hydroxy, dans un support aqueux pour la teinture de fibres, en particulier de fibres kératiniques.

2. Utilisation de nitrophénols substitués par pyrrolyle selon la revendication 1, **caractérisée en ce qu'**un des substituants R², R³, R⁴ ou R⁵ représente un groupe nitro et les autres de ces substituants représentent hydrogène.

3. Utilisation de nitrophénols substitués par pyrrolyle selon la revendication 1, **caractérisée en ce que** R² représente hydrogène ou chlore.

4. Utilisation de nitrophénols substitués par pyrrolyle selon l'une quelconque des revendications 1 à 3, **caractérisée en ce que** R⁶ représente hydrogène.

5. Utilisation de nitrophénols substitués par pyrrolyle selon la revendication 1, **caractérisée en ce que** les composés selon la Formule (I) sont choisis parmi le 4-nitro-2-(1H-pyrrol-1-yl)phénol, le 3-nitro-4-(1H-pyrrol-1-yl)-phénol, le 2-nitro-4-(1H-pyrrol-1-yl)phénol, le 4-nitro-6-chloro-2-(1H-pyrrol-1-yl)-phénol, le 3-nitro-6-(1H-pyrrol-1-yl)phénol.

6. Agent pour la teinture et la coloration de fibres kératiniques, en particulier de cheveux humains, **caractérisé en ce qu'**il contient dans un support aqueux, comme colorant montant directement sur la fibre, au moins un nitrophénol substitué par pyrrolyle de formule (I).

7. Agent selon la revendication 6, **caractérisé en ce que** les nitrophénols substitués par pyrrolyle de formule (I) sont contenus en une quantité de 0,01 à 5% en poids.

8. Agent selon l'une quelconque des revendications 6 ou 7, **caractérisé en ce qu'**au moins un autre colorant montant directement sur la fibre et/ou au moins un précurseur d'un colorant d'oxydation est contenu en plus des nitrophénols substitués par pyrrolyle de formule (I).

9. Agent selon l'une quelconque des revendications 6 à 8, **caractérisé en ce qu'**au moins un dérivé d'indole et/ou d'indoline y est contenu.

10. Agent selon l'une quelconque des revendications 6 à 9, **caractérisé en ce que** le support est une crème, une émulsion, un gel, un shampooing ou un aérosol de mousse.

11. Composés de formule (I), **caractérisés par** les combinaisons de substituants A et B suivantes
| | R¹ | R² | R³ | R⁴ | R⁵ | R⁶ |
|---|---|---|---|---|---|---|
| A | H | -NO₂ | -OH | H | H | H |
| B | -OH | Cl | H | -NO₂ | H | H |
